# NEW EUROPEAN PATENT SPECIFICATION

(11) **EP 0 127 958 B2**
(45) Date of publication and mention of the opposition decision: **10.04.1996**
(45) Mention of the grant of the patent: 11.03.1992
(21) Application number: 84303091.7
(22) Date of filing: 08.05.1984
(51) Int. Cl.: G01N 33/48

(54) **Sensor electrode systems**
Sensor-Elektroden
Electrodes pour des détecteurs

(30) Priority: 05.05.1983 GB 8312262; 05.05.1983 GB 8312261; 06.09.1983 GB 8323799; 16.12.1983 GB 8333644; 11.01.1984 GB 8400650; 29.02.1984 GB 8405262; 29.02.1984 GB 8405263
(43) Date of publication of application: 12.12.1984
(60) Divisional application: 89116797.5
(73) Proprietor: MediSense, Inc., Cambridge Massachusetts 02139 (US)
(72) Inventor: Higgins, Irving John, Ravensden-Bedford MK44 2TF (GB); McCann, James Michael, London W14 0ES (GB); Davis, Graham, Plainsboro-NJ 08536 (US); Hill, Hugh Allen Oliver, Oxford OX2 9JH (GB); Zwanziger, Ron, Brookline, MA 02146 (US); Treidl, Bernhard Ludwig, Boston Mass. (US); Birket, Nigel Norman, Sutton Ely Cambridgeshire CB6 2PG (GB); Plotkin, Elliot Verne, Bedford, MK43 SG (GB)
(74) Representative: Dost, Wolfgang, Dr.rer.nat., Dipl.-Chem.

(56) References cited:
- EP-A- 0 078 590
- DE-A- 2 127 142
- DE-A- 3 038 883
- US-A- 3 224 436
- US-A- 3 458 421
- US-A- 4 224 125
- US-A- 4 225 410
- US-A- 4 376 689
- A.P.F. Turner et al. Biochemical Society Transactions 11, 445-448 (1983)
- H.A.O. Hill et al., Biochemical Society Transactions 11, 453-455 (1983)
- "Medical and Biological Applications of Electro-chemical Devices" (1980), pp. 6-11
- F. Oehme, "Chemische Sensoren" Vieweg Verlag (1991)
- C.C. Liu et al., Diabetes Care 5, 275-277 (1982)
- S.J. Updike et al., Nature 214, 986-988 (1967)
- "Marine Technology" (1980) 468-472
- Review IEE Transactions on Biomedical Engineering (1978) 479-481

## Description

THIS INVENTION relates to sensor electrode systems.

Sensor electrode systems are known, for example, from DE-2127142, EP 78590 and US 4224125. The present invention is concerned with sensor electrode systems of the kind generally known from US 4224125. Our European Patent Application 82305597 (EP-A 78636) describes the construction of sensors comprising a conductive electrode coated with a mixture, or layers, of a catalytically active enzyme and a mediator compound and usually further coated with a retaining permeable membrane. When such a coated electrode is contacted with a substrate containing a species for which the enzyme exerts a catalytic effect, the mediator compound transfers charge to the electrode and this can be used to give a readout signal, against a standard electrode, correlated with the concentration of the said species, even in the presence of other species since enzymes are typically highly selective in their catalytic action.

Thus, numerous types of enzyme-coated electrodes have been utilised each specific to the presence of a physiological or other substrate for which the particular enzyme acts as a catalyst and each therefore potentially capable of acting to detect, measure or monitor the level of the substrate in vivo or in vitro and give a readout correlated for instance with an underlying physiological condition controlling or affecting the substrate level. In particular, use of glucose oxidase or bacterial glucose dehydrogenase as the enzyme, associated with suitable electron-transferring mediator compounds, has been shown to give readout signals correlating linearly with in vitro blood glucose levels over a wide range thus giving a diagnostic or measuring tool for diabetic conditions. Also, such electrodes can measure glucose levels in plasma, serum, interstital fluid, saliva or urine.

The mediator compounds described in EP-A 78636 include polyviologens, fluoranil and chloranil. However, the preferred mediator compounds are metallocene compounds, and in particular the ferrocenes (biscyclopentadienyl iron and its derivatives).

The particular advantages of ferrocenes are as follows:-(a) a wide range of redox potentials accessible through substitution of the cylopentadienyl rings (b) functionalisation of the rings, e.g. to confer solubility or chemical linkability to other such rings or other system components (c) electrochemically reversible one-electron redox properties (d) pH -independent redox potential and (e) slow autooxidation of the reduced form.

The ferrocene structure may be modified by substitution on the rings, and/or by association or polymerisation, which modifications affect the physical, chemical and electrical behaviour so that optimisation of a particular sensor electrode material is possible. In general use, the compound 1,1'dimethylferrocene is a valuable mediator.

Copending Application EP 84303090.9 of even date herewith entitled "Assay techniques utilising specific binding agents" is concerned with the effect on the enzyme and/or mediator electro chemical availability of specific binding agents e.g. antigens/antibodies and others. It can be embodied by specialised electrodes. Such electrodes fall within the scope of the present invention and are discussed below. The interested reader is referred to the published specification, EP-A 125139.

The prior art Application EP-A 78636 referred to above discloses equipment utilising such sensor electrodes. In general it is suitable for research or institutional (e.g. hospital) use. The present invention is concerned with providing sensor electrodes and equipment for use by lab persons or without extensive technical back-up services.

US-A-4 225 410 describes a sensor electrode system for measuring or monitoring glucose in a liquid mixture by monitoring the anodic current resulting from the electro-oxidation of hydrogen peroxide produced when a glucose oxidoreductase enzyme catalyses a redox reaction of glucose. Alternatively, anodic charge is measured. The sensor electrode system comprises a throw away test electrode carrier carrying an area of working electrode material comprising the glucose enzyme, the area being adjacent to but non-contiguous with an area of reference electrode material, both electrode areas being of small dimension and extending as or supported on the electrode carrier to facilitate manipulation before or during contact with a small withdrawn sample of blood.

US-A-4 376 689 describes enzyme electrodes which contain an oxidore-ductase enzyme and a corresponding coenzyme, such as NAD⁺ or NADP⁺, in addition to an electron-conducting material, such as graphite. The coenzyme serves as a transferor of electrons between enzyme and electrodes and vice versa.

*"Medical and Biological Applications of Electro-chemical Devices"* (1980), pages 7 - 11, contains general comments concerning the application of methods termed amperometry, voltammetry and polarography in medicine and biology.

F. Oehme, *"Chemische Sensoren",* Vieweg Verlag (1991), pages 76/77, contains a statement that amperometry is a method belonging to voltammetry.

C.C. Liu et al., Diabetes Care 5, 275 - 277 (1982) describes a miniaturized sensor for determining oxygen wherein a reference electrode of silver/silver chloride is located adjacent to a working electrode made from gold on a flat elongate carrier material made from aluminum. The possibility of using such oxygen sensors as throw away sensors is addressed.

S.J. Updike et al., Nature 214, 986 - 988 (1967) is cited as a reference in C.C. Liu et al. for the proposition that measurements of glucose in blood or extracellular fluid can be accomplished by measuring the oxygen levels in the presence of an enzymatic catalyst, glucose oxidase, by using two oxygen sensors.

*"Marine Technology",* (1980), pages 468 - 472 describes a dissolved oxygen sensor comprising an anode of the silver/silver chloride type and a cathode of the gold type used for obtaining data in numerous potential biological applications and particularly in oceanographic applications.

Use may be made of such electrodes in chemical industry especially where complex mixtures are encountered, e.g. in food chemistry or biochemical engineering. They are however of particular value in biological investigation or control techniques, in human or animal medicine.

We have now established certain design criteria in the production of such electrodes for lay, or clinic. use.

The electrodes can be used in an invasive probe( i.e. one which enters body tissue to contact a body fluid such as whole blood or subcutaneous tissue fluid) or as part of an external test upon a withdrawn sample (using a syringe) or upon an expressed sample (e.g. using a needle-prick device). In each instance the electrode must be as small as practical to avoid trauma either on invasion of the tissue or withdrawing of the sample.

It must be elongate, either to fit within a pointed needle, or for ready handling as an electrode for ready assembly to equipment on the one hand and contact with the sample on the other. It must be sensitively manipulable. It must carry, prior to assembly or in the assembly structure, the reference electrode as well as the 'sensitive' electrode, in spaced non-contiguous relationship.

The present invention provides a sensor electrode system of the kind for use in measuring or monitoring glucose in a liquid mixture, by monitoring the flow of current when a glucose oxidoreductase enzyme catalyses a redox reaction of glucose, and a mediator compound transfers electrons between an electrode and the catalytically active oxidoreductase enzyme; characterised in that the sensor electrode system comprises a throw a way strip test electrode carrier carring an area of working electrode material comprising the glucose enzyme and ferrocene or a ferrocene derivative at the mediator compound, the area being adjacent to but non-contiguous with an area of reference electrode material, both electrode areas being of small dimension, and extending as or supported on the electrode carrier formed as an elongate member to facilitate manipulation before or during contact with live tissue or with a small withdrawn sample or body fluid, and wherein said working electrode material is a single layer admixture of said glucose enzyme, said ferrocene mediator and conductive carbon.

Sensor electrode systems of this invention can be dipped into or similarly contacted with a liquid substrate e.g. a glucose-containing small blood sample or drop of blood.

In one aspect of the invention said sensor electrode system comprises separate electrical connection to each electrode for attachment to a read-out means denoting amount, or monitored level of glucose in a liquid medium with which the support member is contacted to contact both electrodes.

The elongate carrier conveniently comprises a flat strip.

The first electrode is preferably formed of carbon e.g. a filter paper containing carbon. We have also found that carbon foil e.g. as available under the Trade Marks "GRAPHOIL" or "PAPYEX" is a valuable electrode material. As to the glucose oxidoreductase enzyme, the use of glucose oxidase or dehydrogenase, e.g. the bacterial glucose dehydrogenase from Acinetobacter calcoaceticus is particularly valuable. The mediator compound is ferrocene or a ferrocene derivative (especially 1,1'dimethylferrocene).

By way of example only, carbon foil can be glued to the strip; 1,1'-dimethylferrocene mediator can be deposited on the surface of the foil by evaporation of a toluene solution; and enzyme can be bonded to the surface by the use of 1-cyclohexyl-3-(2-morpholinoethyl) carbodiimide metho-p-toluene sulphonate (referred to below as "carbodiimide").

The second electrode can be any convenient reference electrode. We have found it useful to provide adjacent but not contiguous to the first electrode, a flat layer of silver and to convert the surface thereof to silver chloride so as to give an Ag/AgCl reference electrode.

Typically, the electrical connections can be wires which extend down, and are preferably adhered to, the strip, and make electrical contact each with its respective electrode.

The readout means is preferably a digital indicator suitably connected to a dedicated potentiostat which poises the carbon electrode potential at e.g. +150mV Ag/AgCl for a glucose system. The current flowing is then proportional to glucose concentration.

In a particularly valuable version of this type of sensor, it comprises (a) a flat first electrode area of known area small enough to be completely coverable by the smear of blood produced from a non-expressed drop of blood generated from a needle-prick at a bodily extremity. (b) a reference electrode area on the same surface separate from but sufficiently close to the sensitive electrode area that the said blood smear also reaches the reference electrode to establish electrical communication and (c) conductive elements extending separately along the same surface of,and thus insulated from the elongate support member, communicating one with each electrode for connection to signal read-out means attachable to one end of the member.

The area of the first (i.e. sensitive) electrode is generally substantially square; it may be rectangular or otherwise shaped, but in any case usually will correspond in area to a square of 5 mm edge length, or below e.g. from 2 to 4 mm. Equipment utilising the sensors of this invention can be portable or "desktop".

In one form, equipment utilises such electrodes to give visible readout correlated with a selected physiological parameter thus being capable of use in human or veterinary medicine by medical or nursing personnel, or by experienced lay subjects on a self-measurement basis.

For convenience, this document will refer hereinafter to blood-glucose-measuring equipment as being typical but not limitative of equipment with which the sensor electrode systems of the present invention can be used.

Diabetic subjects need to measure their glucose levels frequently. Hitherto, a common method carried out by the subject personally is a colorimetric test using a blood or urine sample which is applied over a surface area containing a colour-reactive detector chemical, adjacent to a comparison area, to give a colour change which is compared with a chart of colour values as an approximate measure of glucose level.

There are however defects in this method. Firstly, colorimetric changes are quantitatively difficult to assess, especially if the patient has impaired vision as a result of the diabetic condition. Indeed, because of this problem expensive automatic colour comparison equipment may need to be purchased by some subjects for interpreting the test results. Secondly, the blood test. while inherently more accurate than a urine test, needs a large enough sample to cover the test surface. Thirdly, it requires the patient to time the colour development accurately. Since blood samples, on a self-treatment basis are taken from body extremities (fingers, toes, earlobes) they are normally not large enough when obtained by a simple needle-prick, and must in fact be expressed i.e. squeezed or massaged out to form a larger drop. Progressively, the tissue of the extremities becomes scarred and coarsened by such treatment to an extent whereby finding fresh testing sites presents a problem.

In order to embody the invention on a home-diagnostic basis a main object of the present invention in one aspect is as described above the provision of small scale non-traumatic test pieces, as an external test electrode strip capable of using the naturally-arising small blood droplet from a needle-prick tester, without tissue massage. Examples are described in more detail below.

These small-scale electrodes are intended as single-use throwaway articles and are utilised in conjunction with electrical circuitry and a readout means, to which they must be easily attachable and detachable. Such circuitry and readout means is itself preferably embodied on a very small scale.

We have accordingly found that the totality of the equipment is subject to certain design constraints.

Thus it is a further object of the invention in this form that the device should be non-traumatic to the user either physically e.g. if used with its own invasive probe or psychologically by virtue of its appearance.

It is a further object of the invention in this form that the device should be capable, despite the small size of the throwaway electrode and of the permanent circuitry/readout components, of easy assembly and disassembly even by juvenile or elderly lay users.

It is still a further object of the invention in this form to ensure that the relatively expensive permanent circuitry/readout components should, despite their small size, be of a form which minimizes loss or damage.

It is still a further object of the invention in this form to provide a device the display readings of which are visible and understandable to a non-expert user.

We have now found that these and other objects of the invention can be met by assembly of the circuitry/readout components into a housing resembling a pen or digital-watch.

Thus, for use with sensor electrode systems of the present invention, there is provided an assembly of circuitry and display means for use in producing a readout value as a diagnostic aid in human or veterinary medicine, housed in a pen-like hollow elongate housing having (a) at one end an electrically conductive socket suitable to receive the outer end of the sensor electrode system capable of producing an electrical signal correlating with a physiological parameter to which the sensor electrode system is selectively sensitive and (b) towards the other end a digital read-out window for exhibiting a numerical value corresponding to the parameter. A thermistor may also be used for temperature compensation.

The person skilled in the art of designing medical equipment will appreciate that use of the invention extends to the pen-like assembly in combination with an attached sensor electrode system as test member, and to the combination as a kit of interrelated parts of such an assembly with a plurality of test members suitable for one-off use.

The term "pen-like" is a general limitation on size and shape. In functional terms, its characteristics are such that it can be held near the socket between the thumb and the nearer one or two opposed fingers, with the elongate body resting on and extending beyond the forefinger, but not to an extent that prejudices fine control of the socket end by the thumb and fingers. In numerical terms it can be from 10 to 30 cm. long and from 0.5 to 3 cms across its maximum transverse dimension; more usually it will be from 12 to 20 cms. long and from 0.8 to 1.5 cms. across. It can be generally circular, or polygonal, in cross-section. Each detachable test member is usually a small-scale enzyme-coated sensor electrode, of the type discussed in the earlier Patent Applications listed above, where the enzyme is specifically glucose-catalyzing whereby diabetic conditions can be measured. It may be a flat external strip electrode dimensioned to operate on a small, non-expressed, blood droplet. The socket arrangement will vary accordingly.

In one embodiment of the present invention, two or more sensor electrodes may be incorporated into a single test member. Again, the socket arrangement will vary accordingly.

The readout means will typically be a conventional seven-segment display window towards the rearward end of the "pen" as in conventional pen/watches. In the case of the multiple sensor embodiment described in the preceding paragraph the display may be switchable between each sensor's discrete monitoring circuit, both the display and a single monitoring circuit may be switchable between sensors, or, a specific display may be provided for each of the sensors present.

Such equipment is of course particularly adapted for use with the non-invasive strip sensor defined above.

Another form of equipment for use with the sensor electrode systems of the present invention is so-called "desk-top" equipment, i.e. for general but skilled use in a general clinic.

In this aspect, the aim is to provide suitable equipment for a practitioner of "desk-top" scale and complexity.

In the operation of a glucose sensor a number of relevant technical points and advantages should be considered. These features are:-

### I Constructional Features

### (a) Membrane cover for electrode

Although the enzyme electrode should be in electrical contact with the liquid, it may be found valuable to exclude the sensor from interfering contact with larger molecules or tissue fluid components. This can be done by a covering or surrounding membrane, depending on electrode geometry. Heat-shrinkable thin polymer tubing can be used as, or in connection with, such membranes.

The membranes can be polymerised in situ (e.g. cellulose acetate). A particular valuable membrane is formed by polycarbonate, especially those polycarbonates sold under the Trade Marks "NUCLEOPORE" or "STERILIN". When tissue fluids are examined they may contain ascorbate; polycarbonate membranes do not permit the passage of ascorbate and thus virtually eliminate interference from that substance. Alternatively a polyurethane membrane may be employed.

### (b) Type of carbon

Carbon foil, as strips, or carbon attached to metal meshes, of pyrolytic grade and known by the Trade Marks "GRAPHOIL" and "PAPYEX" are much preferred for carbon-ferrocene electrodes for use with glucose oxidase. Oxygen interference is minimal, there being less than 4% change in signal between anaerobic and fully aerobic samples. Their physical nature is also very convenient for fabrication, especially of small-scale devices.

### II Operational features

### (a) Operational potential

Preferably operation should take place at a potential equivalent to +50 to +200 mV vs. SCE since interference caused by oxidation of other chemical species present is thereby reduced.

### (b) Concentration range

Glucose oxidase can be used to monitor glucose concentrations of 0 to 40 mM, and glucose dehydrogenase at 0 to 20 mM when immobilised on a carbon-ferrocene electrode. The sensor response is linear up to about 40 mM.

### (c) Response times

The glucose oxidase sensor without membrane is kinetically limited giving rapid response times i.e. about 20 seconds to 95% of the steady-state current response.

### (d) Oxygen-sensitivity

Glucose dehydrogenase/ferrocene electrodes are totally oxygen-insensitive.

### (e) Use of third electrode

In practice, a realistic device can achieve good performance without a third electrode, using Ag,AgCl as a reference counter-electrode, as described more fully below.

### (f) pH and temperature

Glucose oxidase electrodes show no change in current output between pH6 and pH9, and are thus relatively pH-insensitive. They are temperature-stable up to 40°C. If necessary temperature compensation can be effected using a thermistor, or a constant temperature jacket may be used. Also, operating with the electrodes diffusion-limited minimises temperature effects.

### (g) Storage of Electrodes

Electrodes may be stored moist. Extended storage, over months or years, may be achieved by freezedrying or air-drying.

Finally, some aspects can be considered of the electrical circuitry for operating the equipment as described.

The sensor electrode systems of this invention can be employed with a measuring device comprising means for comparing an electrical output of the electrode system with an electronic reference and means for providing a signal related to the electrical output of the electrode.

By employing an electronic reference rather than a cell or reference electrode a measurement using a sensor including an electron-transfer electrode may be made without the use of a separate electrode as a reference.

In a preferred embodiment of the invention, the electron-transfer electrode is poised at a fixed potential against a reference electrode, and the current flowing in the electron-transfer electrode is measured.

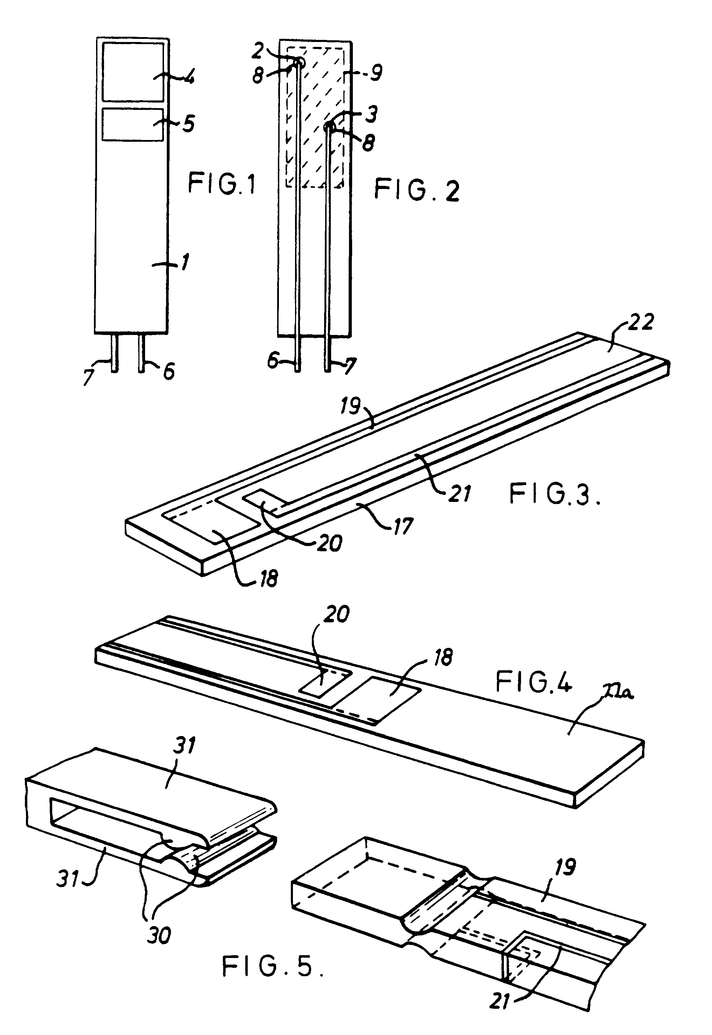

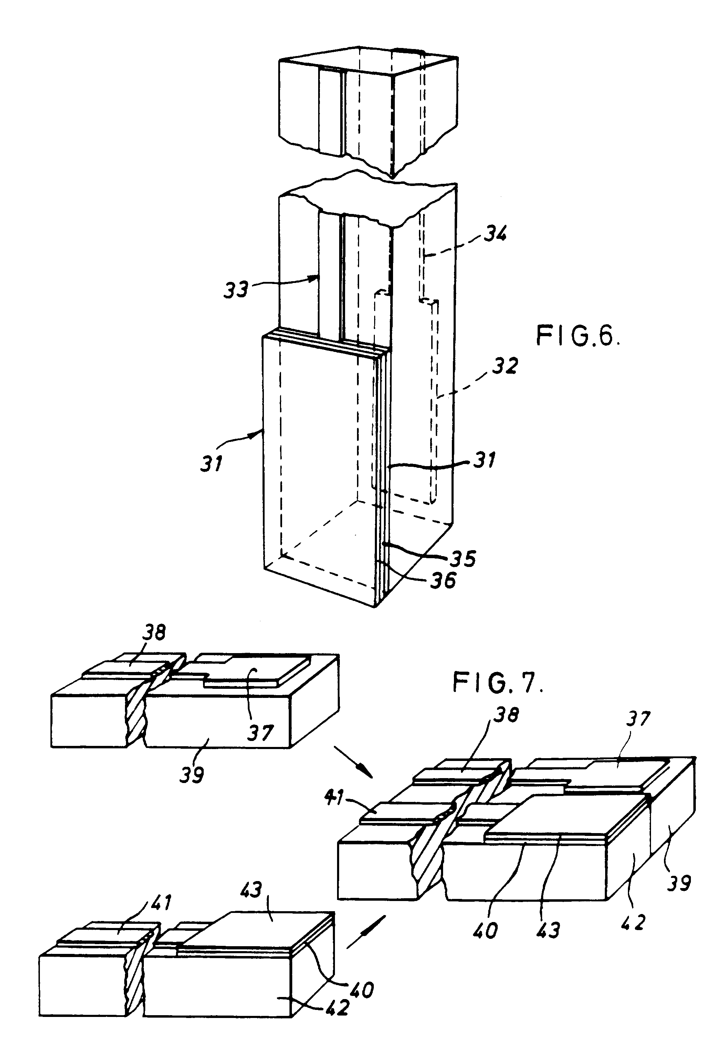

The invention will be further described with reference to the accompanying drawings. In these drawings the actual description of Figures 1/2 and of Figure 6 is not an embodiment in accordance with the invention for the reason that the working electrode material (of electrode 4 in Fig. 1/2 and of electrode 31 in Fig. 6) is not a single layer admixture of glucose enzyme, ferrocene mediator and conductive carbon. However, on the one hand these Figures are helpful in understanding the invention and, on the other hand, the embodiments of Figures 1/2 and 6 represent in fact embodiments of the invention if the electrode construction is carried out as described at the very end of the description under the headline *"Inventive embodiment for the electrode manufacture".*

In the following drawings
Figure 1 is a front view of a strip-supported electrode configuration;
Figure 2 is a back view of the combination shown in Figure 1;
Figure 3 shows an alternative strip-supported electrode;
Figure 4 shows a strip-supported electrode which is a variant of Figure 3;
Figure 5 shows a modified connection of the strip electrode of Figures 3 and 4;
Figure 6 shows a further alternative supported electrode;
Figure 7 shows a combination of two electrode supports;
Figures 8a and 8b are general diagrammatic side views of a pen-like portable holder, of particular utility for the electrodes shown in Figures 3, 4 and 5, having an assembly of circuitry and having a read-out window;
Figure 9 shows a schematic diagram of one form of electrical circuitry for use with the electrodes and equipment of the present invention;
Figure 10 shows a more elaborated circuit diagram for use in the embodiment of Figure 9;
Figure 11 shows a schematic diagram of an alternative embodiment of electrical circuitry; and
Figure 12 shows the more elaborated circuit diagram of a yet further embodiment of circuitry.

In the following description of Figures 1 and 2 dimensions, materials amount and proportions are given by way of example only.

A strip of epoxy glass 1, 9.5 x 40 x 1.6 mm, has two 1 mm diameter holes 2 and 3 therein. A 9 x 9 mm piece of graphite tape or foil 4 is glued on one face, near the end to cover hole 2 and a 4 x 9 mm strip of silver foil 5 is glued adjacent thereto over hole 3. Wires 6 and 7 (Fig. 2) on the back enter holes 2 and 3 respectively for electrical connection with the respective electrode material 4 and 5, being glued in the holes by conductive epoxy resin 8. A stabilising layer of epoxy resin is present over at least part of the back e.g. at 9 to keep the wires in place. Carbon electrode 4 is covered with 1, 1'-dimethylferrocene and glucose oxidase. Silver electrode 5 is covered with silver chloride.

The strip is made up in the following sequence:-
(a) drill holes 2 and 3,
(b) glue on electrodes 4 and 5; "ARALDITE" epoxy resin is suitable but should not enter holes 2 and 3,
(c) attached wires 6 and 7, using conductive epoxy 8, and apply "ARALDITE" resin at 9 the fix the wires in place,
(d) hold the silver electrode at +400 mV vs. SCE in 5M chloride for 10-15 seconds to deposit a thin AgCl layer,
(e) apply a solution of 1,1'-dimethylferrocene (4 µl) in toluene (20 mg/ml) to the graphite tape 4 and allow to evaporate,
(f) cover the ferrocene-coated tape with 50 µl of carbodiimide (25 mg/ml) in pH 4.5 acetate buffer for 1 1/2 hours and
(g) rinse and cover with glucose oxidase (12.5 mg/ml) in pH 5.5 acetate buffer for 2 hours.

The strip can be used by attaching wires 6 and 7 to a potentiostat poising the potential at electrode 4 at +150 mV. vs. Ag/AgCl, and dipping the strip into a glucose-containing solution so that both electrodes 4 and 5 are covered. The shape of the strip facilitates such handling. The current flowing is proportional to glucose concentration.

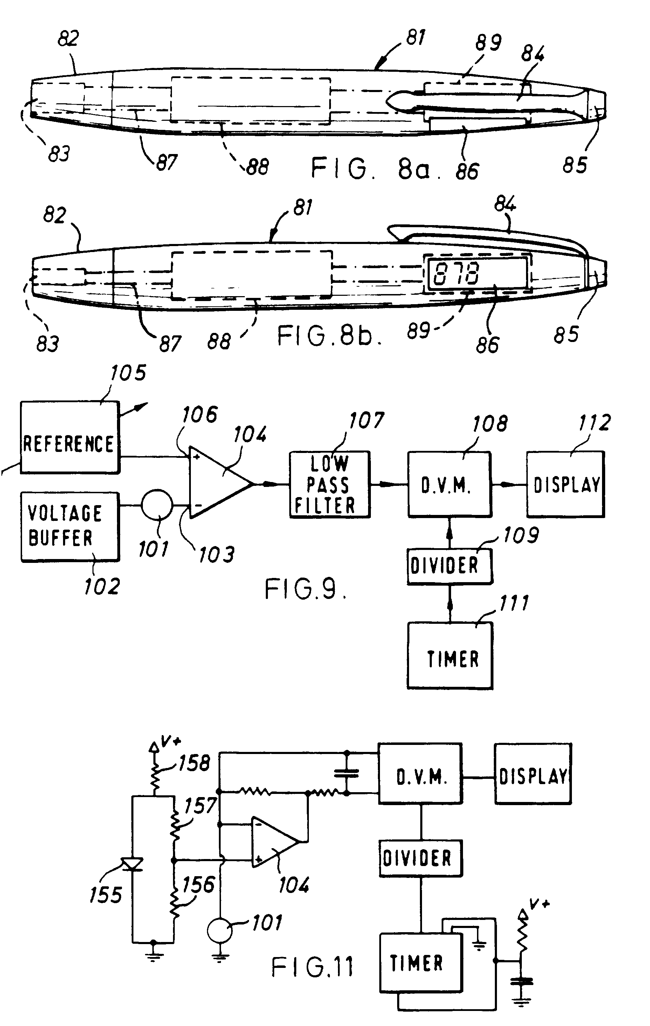

Figure 3 shows a strip electrode 17 made of, for example, a ceramic material or printed-circuit-board laminate. It includes a square area 18 with connector lead 19, the square being covered with the enzyme-containing layers as described above. It further includes a small reference electrode area 20 and separate connector lead 21. The rearward end 22 of the electrode 17 fits into a socket as shown in Figures 8a and 8b and described below. It is to be noted that, as with the needle 10, the electrode strip 17 is a small-scale device. Thus square area 18 is of a side length only about half that of each of two square colorimetric test areas of conventional diagnostic tests and can be used with the original non-expressed bead of blood from a needle-prick device, which is adequate to cover the whole of the square area and communicates electrically with reference electrode area 20.

Modifications may be made to the embodiments shown in Figure 3. For example the strip electrode as shown in Figure 3 can be longer, whereby electrodes 18 and 20 are located only partway along the strip, leaving a free end 22a to facilitate ease of handling by the patient without damaging or touching the electrodes. Also, the electrode strip can clip within two opposed contacts or resilient mounting 31, the routing of one or other of conductive lines 19 and 21 being modified accordingly.

Figure 4 shows a longer strip, and Figure 5 shows the inner end of a strip held between two resilient metal contact strips.

In Figure 6 a 2 cm length of electrically insulating polymer for example MYLAR or TEFLON (a polyfluorocarbon) 0.3 mm square in transverse cross-section is provided with a palladium-silver conductive electrode 31, on the front surface as shown, and a second, smaller electrode 32, on the back as shown in dotted lines. In each case conductive lines 33 and 34 respectively, were formed simultaneously with the electrodes.

On the front electrode 31 is painted a mixture of toluene, 1,1'-dimethyl ferrocene and graphite, formed by mixing a solution of the toluene and 1,1'-dimethylferrocene and a slurry of toluene and graphite. It is believed that the ferrocene is adsorbed on to graphite particles. After drying the mixture forms a layer 35. A layer 36 of glucose oxidase is then immobilised on the graphite surface by carbodiimide immobilisation, known per se (enzyme adsorption can also be used). The electrode may then be covered, on both sides, with a semipermeable membrane of cellulose acetate (or polyurethane), not shown, to block large interfering species from contact with the electrode.

The square section of the support helps in the painting of slurry, or the enzyme-attachment stages, in keeping the electrodes 31 and 32 distinct.

The small scale electrode so produced could be used per se but is especially valuable for incorporation into a standard gauge needle, giving a blood-glucose reading using the same invasive member as the eventual injection.

When producing such small scale needles, the exact sequence of steps can vary. For example, graphite could first be painted on, and a solution of the mediator (ferrocene, etc) in toluene then be applied by dipping into the graphite. Likewise, the enzyme can be applied in solution for adsorption. There is therefore a danger that the reference electrode e.g. silver/palladium could be adversely affected by the solvents or solutes used.

Figure 7 shows the key steps of a procedure which can be used to advantage in the fabrication of these microelectrodes.

The reference electrode 37 and its conductive lead-out strips 38 are formed in silver/palladium on TEFLON base 39. Similarly, an electrode support 40 and lead-out strip 41 are formed in silver/palladium on TEFLON base 42. Only this base 42 and its electrode support are then subjected to (a) painting on a graphite slurry in toluene (b) dipping in 1',1'dimethylferrocene solution in toluene and (c) contacting with the enzyme to absorb e.g. glucose oxidase into the active layer 43. Thereafter the bases 39 and 42 are glued or held, side-by-side, their general rectangular cross-section facilitating such positive location. Back-to back location is also possible.

As before, the finished assembly may be located inside a needle bore, e.g. with extra access portions near the electrode surfaces.

### Incorporation of 1',1'-dimethylferrocene into the electrode

A solution of 1',1'dimethylferrocene in toluene was mixed into a toluene-based slurry of the graphite powder. The mixture was then painted on to the base conductor 41 and allowed to dry at 43. This provided an electrode surface that was electroactive towards glucose oxidase.

These experiments showed that "thick layer"or screen-printing technology could provide a usable base strip which could easily be coated with a stable graphite surface and that moreover the electrode surface could be made electroactive towards glucose by adsorption of a ferrocene directly into the coating mixture. In addition, the reference electrode operated satisfactorily in buffered solutions.

Figures 8a and 8b show a holder which is particularly adapted to utilise electrodes as shown in Figures 3, 4, 5 but which could if necessary utilise electrodes as shown in Figures 1 and 2, and 6 and 7 at least of the various embodiments shown.

From above the holder 81 intentionally resembles a conventional pen/watch as much as possible. It has a forward end 82, possibly rotary to tighten the walls of a flattened socket cavity 83 formed within it. A central join, a clip 84 and a press-button 85 all resemble those of a conventional pen, and digital readout-window 86 is also of a type known in pen/watches.

Inside the holder as shown by dotted lines is connection circuitry 87, possibly printed in situ, battery 88 and operating circuitry 89 behind and manufactured as a unit with the display window 86. The display can be capable of operation only when button 85 is pressed so that extra illumination can be provided if necessary.

The embodiments shown in Figures 8a and 8b especially when used in conjunction with the electrodes of Figures 3 - 5 fulfill the design criteria discussed above for such portable equipment.

The delicate manipulation facilitated by the pengrip (e.g. by thumb and finger) means that the small electrodes e.g. of Figures 3, 4 or 5 can be easily assembled into, or detached from, the socket. A user will always orient the holder with the window 86 visible thus always giving a uniform relative orientation to the socket 83 whereby the rearward ends of the fragile electrodes can be fitted without experiment and damage.

The "pen" format is instinctively picked up after use and safely carried in a pocket, more so than for any other small device. Thus the expensive part of the equipment is safeguarded. Furthermore, it is possible to incorporate a conventional timer circuit into the device thereby fulfilling the actual function of a pen-type watch and providing an audible or visible signal which marks the point in time at which a reading should be taken.

Finally, the display is numerical, clearly visible and if necessary can be supplemented by an illuminating light source.

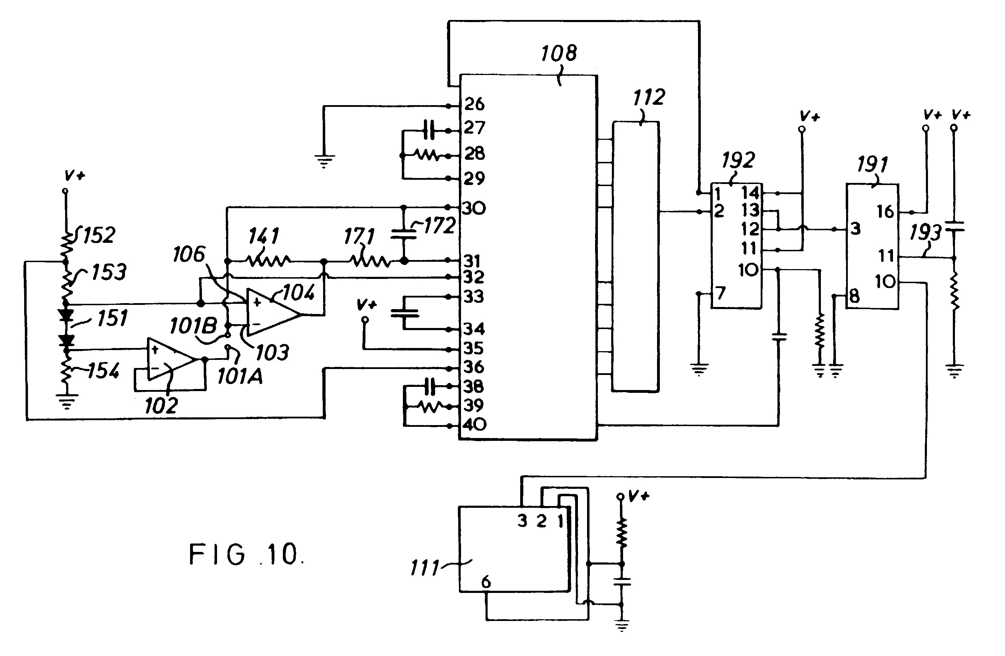

In Figure 9, a sensor 101 is connected between a voltage buffer 102, and the inverting input 103 of the operational amplifier 104 which is configured as a current amplifer. An electronic reference 105 connected to the non-inverting input 106 of the operational amplifier is fed into a low-pass filter 107 which removes rapid signal fluctuations (which may be due to noise, earth hum or other sources of interference) while allowing the filtered output of the operational amplifier 104 to be fed into the digital volt meter (D.V.M.) 108.

The digital volt meter is supplied with clock pulses via the divider 109, from the timer 111. The D.V.M drives a liquid crystal display 112. The electronic reference 105, is either of a pre-selected value or capable of being selected for a particular electron transfer electrode.

In each of the embodiments of electrode discussed above the sensor comprises a mediator-carrying surface which has one or more enzymes immobilized thereupon. The sensor further includes a silver/silver chloride (Ag/AgCl) internal reference electrode. If, for example, a voltage of + 200 mV volts is preferentially dropped across the electrode as is the case with a glucose -oxidase-containing electrode, then the reference voltage 105 is selected accordingly.

In Figure 10. the electronic reference comprises resistors 152, 153 and 154 together with diodes 151. A voltage is selected at 0.3V by a suitable choice of resistor values at 152, 153 and 154. The voltage across the resistor 153 is 1V, which ensures that 100 µA of electrode current will give a full scale reading of 999 on the liquid crystal display 109.

Non-inverting buffer 102 ensures that the voltage on the terminal 101A remains substantially constant The sensor 1 (Figure 1) is connected across terminals 101A and 101B.

The operational amplifier 104 is connected via its inverting input 103 to the terminal 101B and the feedback resistor 141. The non-inverting input 106 is connected to the electronic reference.

A first-order low-pass filter 171, 172 is connected across the feed-back resistor 141, and supplies an analog signal to the D.V.M. 8. Pin values are given for a 7116CPL chip (manufactured by Motorola). The D.V.M. drives a liquid crystal display 112.

Clock pulses for the D.V.M. are supplied from the timer 111, (pin values are given for a 555 chip) via the dividers 191 (pin values for an MC 14020B) and 192 (pin values for an MC14016B chip). The connection 193 to pin 11 of the divider 191 enables a power-up reset.

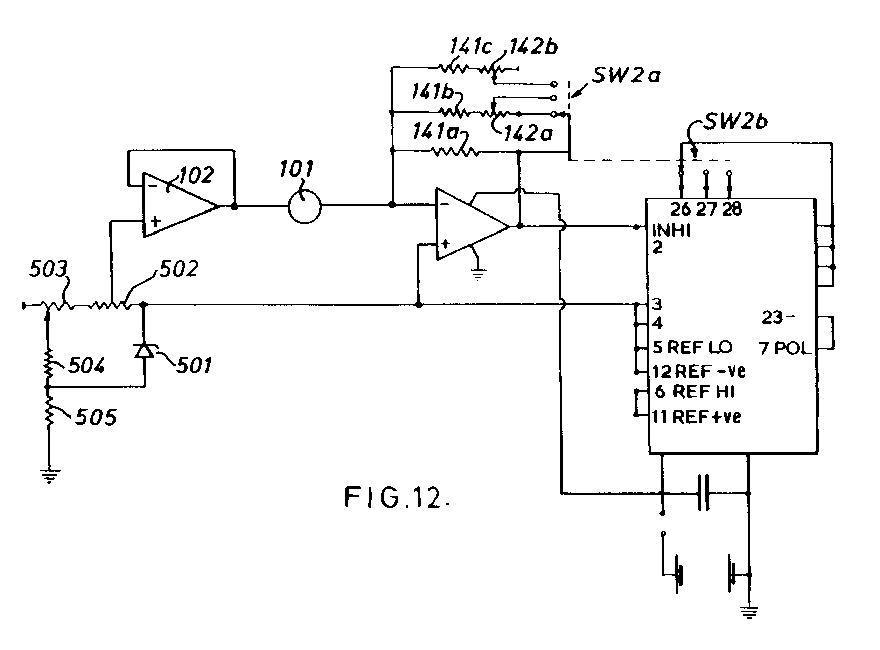

In Figure 11 an alternative circuit is shown which does not make use of the non-inverting voltage buffer 102, but has the sensor 101 connected between the operational amplifier 104, and ground. The embodiment shown in Figure 11 employs a fixed reference voltage which is provided by a circuit differing from that of Figure 10.

In this embodiment, the diode 155 functions as a voltage-reference diode and provides a reference voltage drop across its ends equal to the diode forward voltage.

By a suitable choice of the values of the resistors 156, 157 and 158 the correct voltage may be applied across the electrode. In this embodiment the sensor again employs as a reference an Ag/AgCl couple and immobilized glucose oxidase in the presence of a mediator compound as the electron-transfer electrode.

Figure 12 shows a third embodiment of the present invention, which provides a continuously variable reference voltage which may be selected to accommodate any type of electron-transfer electrode, that is, one, which for example, employs any of the enzymes listed herein or any combination of these enzymes. The LED display is not shown.

In Figure 12 the feedback resistor 141 in circuit at any given time may be selected from resistors 141a, 141b, and 141c by means of switch SW2a which is ganged with switch SW2b. This allows the current output of the sensor 101 to be displayed in three ranges, for example, 1 µA 10 µA or 100 µA full scale. Furthermore, the range may be trimmed by using the variable resistors 142a and 142b.

The embodiment shown in Figure 12 has the non-inverting voltage buffer 102 of the embodiment shown in Figure 10.

The reference voltage for the embodiment shown in Figure 12, is derived from the circuit elements 501-505 which include the potentiometers 503 and 502 providing a variable voltage across the sensor 101, thereby accommodating any type of electron-transfer electrode. It is envisaged that the continuously variable resistors 142a and 142b could be replaced in certain applications by stepwise resistance switching means with each position or setting being dedicated to a particular type of electrode.

Various modifications may be made in the circuitry. For example the liquid crystal display may be replaced by a plotter or a dosage control device, or a temperature stability circuit may be incorporated.

Inventive embodiment for the electrode manufacture.

The electrode is manufactured by screen printing techniques e.g. in a multi-stepped procedure comprising:-
I - screen printing of Ag/AgCI reference electrode and metal tracing.
II - screen printing of the active electrode with a printing ink comprising a colloidal carbon, glucose oxidase in buffer, and an organic polymer.
III - screen printing, spraying or dip coating to provide a membrane over the assembly.

Advantages of this method are that it is amenable to high volume automation, and is of high reproducibility

To this end, there can be used a suspension in a liquid medium of carbon together with (a) glucose oxidase as enzyme and (b) ferrocene or a ferrocene derivative as mediator compound capable of transferring charge to the said carbon from the enzyme when the enzyme is catalytically active, the said suspension being formed as a printable and conductive ink for use in the fabrication of electrodes as described above.

## Claims

1. A sensor electrode system of the kind for use in measuring or monitoring glucose in a liquid mixture by monitoring the flow of current when a glucose oxidoreductase enzyme catalyses a redox reaction of glucose, and a mediator compound transfers electrons between an electrode and the catalytically active oxidoreductase enzyme; characterised in that the sensor electrode system comprises a throw away strip test electrode carrier carring an area of working electrode material comprising the glucose enzyme and ferrocene or a ferrocene derivative as the mediator compound, the area being adjacent to but non-contiguous with an area of reference electrode material, both electrode areas being of small dimension, and extending as or supported on the electrode carrier formed as an elongate member to facilitate manipulation before or during contact with live tissue or with a small withdrawn sample of body fluid, and wherein said working electrode material is a single layer admixture of said glucose enzyme, said ferrocene mediator and conductive carbon

2. A sensor electrode system according to claim 1. comprising separate electrical connection to each electrode for attachment to a read-out means denoting amount, or monitored level of glucose in a liquid medium with which the support member is contacted to contact both electrodes.

3. A sensor electrode system according to claim 2, which comprises (a) a flat first electrode area of known area small enough to be completely coverable by the smear of blood produced from a non-expressed drop of blood generated from a needle-prick at a bodily extremity, and (b) a reference electrode area on the same surface separate from but sufficiently close to the sensitive electrode area that the said blood smear also reaches the reference electrode to establish electrical communication and (c) separate conductive elements extending along the same surface of the elongate support member, communicating one with each electrode for connection to signal read-out means attachable to one end of the member.

4. A sensor electrode system according to claim 1, 2 or 3 in which the area of working electrode material is square.

5. A sensor electrode system according to any of claims 1 to 4, in which the area of working electrode material is less than 25 mm.

6. A sensor electrode system according to any preceding claim, in which the reference electrode is silver/silver choride.

7. A sensor electrode system according to claim 1, wherein the working electrode and the reference electrode are carried on the same face of an elongate non-conductive carrier of rectangular cross-section.

8. A sensor electrode system according to claim 7, in which the area of the working electrode material is towards one end of the surface of the elongate member, and the area of the reference electrode is also towards the said end.

## Patentansprüche

1. Sensor-Elektrodensystem zur Verwendung beim Messen oder Überwachen von Glukose in einer flüssigen Mischung, durch Überwachen des Stomflusses bei einer Redoxreaktion von Glukose, die durch ein Glukose-Oxidoreduktase-Enzym katalysiert wird, wobei eine Vermittlerverbindung Elektronen zwischen einer Elektrode und dem katalytisch aktiven Oxidoreduktase-Enzym überträgt, dadurch gekennzeichnet, daß das Sensor-Elektrodensystem einen wegwerfbaren Streifentest-Elektrodenträger aufweist, der eine Fläche aus Arbeits-Elektrodenmaterial trägt, welche das Glukoseenzym und Ferrocen oder ein Ferrocenderivat als Vermittlerverbindung umfaßt, wobei diese Fläche benachbart zu einer Fläche aus Referenz-Elektrodenmaterial ist, jedoch nicht an diese anstößt, wobei die beiden Elektrodenflächen klein dimensioniert sind und sich als längliches Teil oder als vom länglich geformten Elektrodenträger getragenes Teil erstrecken, um die Handhabung vor oder während eines Kontaktes mit Lebendgewebe oder mit einer kleinen Entnahmeprobe aus Körperflüssigkeit zu erleichtern, und wobei das Arbeits-Elektrodenmaterial ein einschichtiges Gemisch des Glukoseenzyms, der Ferrocen-Vermittlerverbindung und leitfähigem Kohlenstoff ist.

2. Sensor-Elektrodensystem nach Anspruch 1, das eine getrennte elektrische Verbindung zu jeder Elektrode für einen Anschluß an ein das Vorhandensein, die Menge oder den überwachten Pegel der Glukose in einem flüssigen Medium anzeigenden Auslesemittel, mit dem sich der Träger in Kontakt befindet, um beide Elektroden in Kontakt zu bringen, umfaßt.

3. Sensor-Elektrodensystem nach Anspruch 2, mit
(a) einem flachen ersten Elektrodenbereich bekannter Fläche, die klein genug ist, um vollständig durch den Blutabstrich bedeckt zu werden, der von einem nicht herausgedrückten Blutstropfen stammt, welcher durch einen Nadelstich in eine Körperextremität entnommen wird,
(b) einer auf derselben Oberfläche angeordneten Referenz-Elektrodenfläche, die zwar getrennt, aber doch so nah zur empfindlichen Elektrodenfläche liegt, daß der Blutabstrich auch die Referenz-Elektrode erreicht, um eine elektrische Verbindung herzustellen und
(c) getrennten, leitfähigen Elementen, die sich entlang der gleichen Oberfläche des länglichen Trägerteiles erstrecken, wobei jeweils eines mit jeder Elektrode für einen Anschluß an Signal-Auslesemittel, die an einem Ende des Teiles angebracht werden können, verbunden ist.

4. Sensor-Elektrodensystem nach Anspruch 1, 2 oder 3, dadurch gekennzeichnet, daß die Fläche aus Arbeits-Elektrodenmaterial quadratisch ist.

5. Sensor-Elektrodensystem nach einem der Ansprüche 1 bis 4, dadurch gekennzeichnet, daß die Fläche aus Arbeits-Elektrodenmaterial kleiner als 25 mm ist.

6. Sensor-Elektrodensystem nach einem der vorstehenden Ansprüche, dadurch gekennzeichnet, daß die Referenz-Elektrode aus Silber/Silberchlorid ist.

7. Sensor-Elektrodensystem nach Anspruch 1, dadurch gekennzeichnet, daß die Arbeits-Elektrode und die Referenz-Elektrode auf derselben Fläche eines länglichen nichtleitfähigen Trägers rechteckigen Querschnittes aufgebracht sind.

8. Sensor-Elektrodensystem nach Anspruch 7, dadurch gekennzeichnet, daß die Fläche des Arbeits-Elektrodenmaterials zu einem Ende der Oberfläche des länglichen Teiles hin angeordnet ist, und daß die Fläche der Referenz-Elektrode ebenfalls zu diesem Ende hin angeordnet ist.

## Revendications

1. Un système d'électrodes pour détecteurs du genre destiné à une utilisation dans la détection, la mesure ou le contrôle de glucose dans une mélange liquide en contrôlant le débit de courant lorsqu'une enzyme oxydoréductase de glucose catalyse une réaction rédox de glucose et qu'un composé médiateur transfère des électrons entre une électrode et l'enzyme oxydoréductase catalytiquement active, caractérisé en ce que le système d'électrodes pour détecteurs comprend un porteur d'électrode sous forme d'une languette test à jeter comportant une zone de matière formant électrode de travail comprenant l'enzyme de glucose et le ferrocène ou un dérivé de ferrocène comme le composé médiateur, la zone étant adjacente mais non contiguë à une zone de matière formant électrode de référence, les deux zones d'électrodes étant de faible dimensions et s'étendant sur ou supportées par le porteur d'électrodes qui se présente sous la forme d'un élément allongé de manière à faciliter la manipulation avant ou pendant le contact avec le tissu vivant ou avec un petit échantillon prélevé de fluide corporel, dans lequel ladit matière formant électrode de travail est un mélange de ladite enzyme de glucose, ledit ferrocène et du carbone conducteur.

2. Un système d'électrodes pour détecteurs suivant la revendication 1, comprenant une connexion électrique séparée à chaque électrode pour un raccordement à un dispositif de sortie des données de mesure montrant, la quantité ou le taux contrôlé de la glucose dans un milieu liquide avec lequel l'élément support est mis en contact de manière à assurer le contact avec chacune des électrodes.

3. Un système d'électrodes pour détecteurs suivant la revendication 2, qui comprend
(a) une première zone d'électrode plane de zone connue suffisamment petite pour pouvoir être complètement couverte par la tache de sang produite par une goutte non exprimée de sang provenant d'une piqûre d'aiguille à une extrémité corporelle et
(b) une zone d'électrode de référence sur la même surface mais séparée de tout en étant suffisamment proche de la zone d'électrode sensible de sorte que ladite tache de sang atteint également l'électrode de référence pour établir une communication électrique et
(c) des éléments conducteurs séparés s'étendant le long de la même surface de l'élément support allongé, communiquant chacun avec une électrode pour assurer la connexion avec un dispositif de réception des données de mesure pouvant être attaché à une extrémité du membre.

4. Un système d'électrodes pour détecteurs suivant la revendication 1, 2 ou 3, dans lequel la zone de la matière formant électrode de travail est carrée.

5. Un système d'électrodes pour détecteurs suivant l'une quelconque des revendications 1 à 4, dans lequel la zone de la matière formant électrode de travail est inférieur à 25 mm.

6. Un système d'électrodes pour détecteurs suivant l'une quelconque des revendications précédentes, dans lequel l'électrode de référence est une électrode argent/chlorure d'argent.

7. Un système d'électrodes pour détecteurs suivant la revendication 1, dans lequel l'électrode de travail et l'électrode de référence sont supportées sur la même face d'un support allongé non conducteur de section transversale rectangulaire.

8. Un système d'électrodes pour détecteurs suivant la revendication 7, dans lequel la zone de la matière formant électrode de travail est située vers une extrémité de la surface de l'élément allongé et la zone de l'électrode de référence est également située vers la même extrémité.
